# EUROPEAN PATENT APPLICATION

(11) **EP 4 714 433 A1**
(43) Date of publication of application: **25.03.2026**
(21) Application number: 24862114.6
(22) Date of filing: 09.09.2024
(51) Int. Cl.: A61K 9/12, A61K 9/14, A61K 31/4035, A61K 31/44, A61K 31/437, A61K 31/69, A61K 31/421, A61K 47/02, A61K 47/26, A61K 47/10, A61K 47/06, A61P 11/00

(54) **INHALATION FORMULATION FOR TREATING IPF DISEASE AND PREPARATION METHOD THEREFOR**

(30) Priority: 07.09.2023 CN 202311151725
(71) Applicant: Suzhou Inhal Pharma Co., Ltd., Suzhou, Jiangsu 215000 (CN)
(72) Inventor: SHI, Kaiqi, Suzhou, Jiangsu 215000 (CN); ZHANG, Qingzhen, Suzhou, Jiangsu 215000 (CN); WANG, Fangyan, Suzhou, Jiangsu 215000 (CN); LIU, Xunxun, Suzhou, Jiangsu 215000 (CN); YANG, Riwei, Suzhou, Jiangsu 215000 (CN); LIU, Xiujie, Suzhou, Jiangsu 215000 (CN); MA, Yingliang, Suzhou, Jiangsu 215000 (CN); ZHU, Jingxu, Suzhou, Jiangsu 215000 (CN)
(74) Representative: FRKelly
(86) International application number: PCT/CN2024/117709
(87) International publication number: WO 2025/051276

(57) **Abstract**

An inhalation formulation for treating an IPF disease. The inhalation formulation is an aerosol inhalation liquid formulation, an inhalation aerosol, a dry powder inhaler or a soft mist inhaler. The inhalation formulation contains an active drug, and the active drug is any one of apremilast, ibudilast, roflumilast, crisaborole, and difamilast. A preparation process for the inhalation formulation is introduced into a conventional formulation. An active component in a conventional oral formulation is prone to being destroyed in the digestive tract, and has low bioavailability, a liver first pass effect, and a slow onset of action. An inhalation formulation capable of significantly alleviating IPF is prepared. Tests prove that the prepared inhalation formulation can effectively increase the bioavailability, and improve the clinical use effect.

## Description

### TECHNICAL FIELD

The present invention relates to the technical field of inhalation agents, and specifically to an inhalation formulation for treating an IPF disease and a preparation method therefor.

### BACKGROUND

Idiopathic pulmonary fibrosis (IPF) is a non-cancerous lung disease characterized by the formation of scar tissue in the lungs without any known mutagenesis, chronic progressive fibrosis, accompanied by an unstoppable decline in lung function, progressive respiratory failure, and high mortality. IPF is a rare disease that is usually progressive and causes severe disability, affecting approximately 50,000 people worldwide. Currently, the recommended drugs for treating IPF are pirfenidone and nintedanib, but these two drugs have limited efficacy in preventing disease progression and improving quality of life, and are also associated with tolerability issues.

Current IPF treatments are applied orally, while some compounds under development are administered by subcutaneous or intravenous injection.

Despite numerous studies, there are currently no inhalation formulations specifically designed for clinical use in the treatment of IPF.

### SUMMARY OF THE INVENTION

To address the problems existing in the prior art, the present invention provides an inhalation formulation for treating an IPF disease and a preparation method therefor. The prepared inhalation formulation features simple production process, portability, mild respiratory irritation, and improved bioavailability.

The present invention is implemented through the following technical solutions:

Provided is an inhalation formulation for treating an IPF disease, wherein the inhalation formulation is an aerosol inhalation liquid formulation, an inhalation aerosol, a dry powder inhaler, or a soft mist inhaler;
the inhalation formulation contains an active drug, and the active drug is any one of apremilast, ibudilast, crisaborole, and difamilast.

Further, the active drug includes, but is not limited to, apremilast, ibudilast, crisaborole, and difamilast.

Furthermore, the inhalation formulation is an inhalation liquid formulation.

Furthermore, the inhalation formulation is an aerosol inhalation liquid formulation.

Furthermore, the aerosol inhalation liquid formulation consists of an active drug and a pharmaceutical excipient A;
the pharmaceutical excipient A includes a solvent, a nonionic surfactant, a pH buffers, an osmotic pressure regulator, and a complexing agent.

Furthermore, the solvent is deionized water;
furthermore, the nonionic surfactant includes, but is not limited to, Tween, Span, polyol, and poloxamer;
the pH buffer includes, but is not limited to, acetic acid-sodium acetate, phosphate-sodium phosphate, or citrate-sodium citrate, with a pH value of 4.0-6.0;
the osmotic pressure regulator includes, but is not limited to, sodium chloride, propylene glycol, glycerol, and glucose;
the complexing agent is EDTA or a salt thereof.

Furthermore, the nonionic surfactant is any one of Tween, Span, polyol, and poloxamer.

Furthermore, provided is a method for preparing the aerosol inhalation liquid formulation, comprising the following preparation steps:
step S1. dissolving the osmotic pressure regulator and complexing agent in the solvent, stirring the mixture until completely dissolved, adding the active drug, and stirring the mixture until completely dissolved to give a solution A;
step S2. adding the solvent to the nonionic surfactant and well dispersing the system to give a solution B;
step S3. adding the solution B to the solution A, stirring the mixture well, then adding the remaining solvent, and adding the pH buffer to adjust the pH to give the formulation.

Furthermore, the aerosol inhalation formulation is used in an aerosol inhalation device, and the aerosol inhalation device includes, but is not limited to, Aerogen^{®} (vibrating mesh nebulization), Omron^{®} (air compression nebulization), Yuwell^{®} ( ultrasonic nebulization), etc.

Furthermore, the inhalation formulation is an inhalation aerosol.

Furthermore, the inhalation aerosol consists of an active drug component and a pharmaceutical excipient B;
the pharmaceutical excipient B includes a hydrofluoroalkane propellant, a polyethylene glycol surfactant, and a co-solvent.

Furthermore, the active drug component is prepared by micronization, wherein micronization process includes, but is not limited to, air jet milling, spray drying, spray freeze drying, recrystallization, and ball milling.

Furthermore, the hydrofluoroalkane propellant includes, but is not limited to, 1,1,1,2,3,3,3-heptafluoropropane or 1,1,1,2-tetrafluoroethane;
polyethylene glycol includes, but is not limited to, PEG200, PEG400, and PEG 1000;
the co-solvent includes, but is not limited to, propylene glycol or ethanol.

Furthermore, the active drug accounts for 1-3% w/w of the total amount of the formulation;
the polyethylene glycol surfactant accounts for 0.01-1% w/w of the total amount of the formulation.

Furthermore, provided is a method for preparing the inhalation aerosol, wherein the micronized active drug component is added to an aluminum can, the system is weighed, the surfactant and the co-solvent are added, the aluminum can is quickly sealed with a metering valve, the propellant is added through the metering valve, and then the filled aerosol can is sonicated for 30 s to give an inhalation aerosol.

Furthermore, the inhalation aerosol is used in a device for the inhalation aerosol, and the device for the inhalation aerosol includes, but is not limited to, Ventolin^{®}, Lixinping^{®}, and Jishu^{®}, etc.

Furthermore, the inhalation formulation is a dry powder inhaler.

Furthermore, the dry powder inhaler consists of an active drug and a carrier;
the carrier includes, but is not limited to, lactose, mannose, chitosan, DPPC, and DSPC.

Furthermore, the content ratio of the active drug to the carrier is (5-100):(1-95).

Furthermore, the particle size D90 of the active drug is 1-10 µm;
the particle size D90 of the carrier is 3-200 µm.

Furthermore, provided is a method for preparing the dry powder inhaler, comprising the following steps:
S01. micronizing the active drug to give a first component;
S02. mixing the first component with the carrier to give a second component;
S03. preparing the second component into a dry powder formulation and loading it into an appropriate device for later use.

Furthermore, the micronization process includes, but is not limited to, air jet milling, spray drying, spray freeze drying, recrystallization, and ball milling;
the mixing method includes, but is not limited to, high-shear mixing, air jet mixing, and three-dimensional mixing.

Furthermore, the device in S03 includes, but is not limited to, Spiriva^{®}, Seretide ^{®}, Symbicort turbuhaler^{®}, or breezhaler^{®}.

Furthermore, the inhalation formulation is a soft mist inhaler.

Furthermore, the soft mist inhaler consists of an active drug component and a pharmaceutical excipient C;
the pharmaceutical excipient C includes a solvent, an additive and a pH adjuster.

Furthermore, the active drug component is prepared by micronization, wherein micronization process includes, but is not limited to, air jet milling, spray drying, spray freeze drying, recrystallization, and ball milling.

Furthermore, the solvent is deionized water;
the additive is EDTA or a salt thereof;
the pH adjuster is citric acid, hydrochloric acid, or sodium hydroxide, and the pH range of the formulation is 2 to 6.

Furthermore, the concentration of the active drug is 1ug/100mL-1g/100mL;

Furthermore, provided is a method for preparing the soft mist inhaler, wherein the additive and pH adjuster are added to a beaker, a portion of the solvent is added to dissolve them, then the micronized active drug is added, and the solvent is replenished to reach the required concentration, the mixture is well stirred, then the pH of the mixture is measured, and the mixture is put into flexible plastic vials for later use.

Furthermore, the soft mist inhaler is administered via a soft mist device, and the soft mist device includes, but is not limited to, Spiriva Respimat^{®}, etc.

The beneficial effects of the present invention are as follows:
1. According to the present invention, a preparation process for the inhalation formulation is introduced into a conventional formulation. An active component in a conventional oral formulation is prone to being destroyed in the digestive tract, and has low bioavailability, a liver first pass effect, a slow onset of action, and severe systemic side effects. The present invention prepares an inhalation formulation capable of significantly alleviating IPF. Tests prove that the prepared inhalation formulation can effectively increase the bioavailability, and improve the clinical use effect.
2. Currently, the only two drugs that are the first-line treatment for IPF in clinical practice are nintedanib and pirfenidone. In the present invention, we demonstrated the effectiveness of PDE inhibitors in alleviating and improving pulmonary fibrosis in patients with IPF. Furthermore, we modified the drug into an inhalation formulation, avoiding many drawbacks of oral formulations in the treatment of lung diseases, demonstrating good inventiveness.

### BRIEF DESCRIPTION OF THE DRAWINGS

The drawings are provided to further explain the present invention and form part of the specification. They are used together with the embodiments of the present invention to explain the present invention and do not constitute a limitation thereon. In the drawings:
Fig. 1 shows the detection of weight of mice in each group;
Fig. 2 shows MASSON staining of the lungs of mice in each group;
Fig. 3 shows HE staining of the lungs of mice in each group;
Fig. 4 shows the detection of MDA and HYP content in the lungs of mice in each group;
Figs. 5 and 6 show the detection of weight of mice in each group in more groups of examples and comparative examples;
Fig. 7 shows MASSON staining of mice in each group in more groups of examples and comparative examples;
Fig. 8 shows HE staining of mice in each group in more groups of examples and comparative examples;
Fig. 9 shows the detection of content of MDA and HYP of mice in each group in more groups of examples and comparative examples.

### DETAILED DESCRIPTION OF THE INVENTION

The technical solutions of the present invention will be further described in detail below with reference to specific examples, but the scope of protection of the present invention is not limited to the following examples.

IPF (idiopathic pulmonary fibrosis) is a different disease from chronic obstructive pulmonary disease (COPD), asthma, chronic bronchitis, and cystic fibrosis. IPF, short for idiopathic pulmonary fibrosis, is a chronic, progressive, fibrotic interstitial lung disease. This disease is characterized by the replacement of normal lung tissue with scar tissue (fibrosis), which causes the lung structure to gradually harden and thicken, thereby affecting the ability to exchange gases and making it difficult for patients to breathe. The exact cause of IPF is unknown, hence the name "idiopathic," but it may be related to genetic factors, long-term smoking, certain viral infections, gastroesophageal reflux, environmental factors, and abnormal responses of the immune system.

The following are descriptions of several specific examples of the present invention for treating the IPF disease, as well as comparative examples and experimental examples:

### Example 1

The aerosol inhalation liquid formulation of this example includes an active drug component, a solvent, a nonionic surfactant, a pH buffer, an osmotic pressure regulator, and a complexing agent.

The active drug component is roflumilast, with a dose of 20 µg/mL;
the solvent is water;
the nonionic surfactant is Tween 80, with a dose of 100 mg/L;
the pH buffer is phosphate-sodium phosphate with a pH of 4.0;
the osmotic pressure regulator is sodium chloride, with a dose of 9 mg/mL;
the complexing agent is EDTA-2Na, with a dose of 200 mg/L.

The osmotic pressure regulator sodium chloride and the complexing agent EDTA-2Na were dissolved in 1/3 volume of the solvent (water), the mixture was stirred until dissolved, then micronized roflumilast was added and the mixture was stirred until dissolved; the nonionic surfactant Tween 80 was added to 1/3 volume of the solvent (water), the mixture was evenly dispersed and then added to the above solution, the system was well stirred, then the solvent (water) was added to make up to the total volume, and the buffer was added to adjust the pH to 5.0 to give the formulation.

The micronization process in the preparation process is air jet mixing;
the feed pressure of air jet milling is 6 bar, and the discharge pressure of air jet milling is 6 bar.

### Example 2

The aerosol inhalation liquid formulation of this example includes an active drug component, a solvent, a nonionic surfactant, a pH buffer, an osmotic pressure regulator, and a complexing agent, etc.

The active drug component is apremilast, with a dose of 20 µg/mL;
the solvent is water;
the nonionic surfactant is Tween 80, with a dose of 100 mg/L;
the pH buffer is phosphate-sodium phosphate with a pH of 4.0;
the osmotic pressure regulator is sodium chloride, with a dose of 9 mg/mL;
the complexing agent is EDTA-2Na, with a dose of 200 mg/L.

The method for preparing the apremilast aerosol inhalation liquid formulation is as follows:
The osmotic pressure regulator sodium chloride and the complexing agent EDTA-2Na were dissolved in 1/3 volume of the solvent (water), the mixture was stirred until dissolved, then micronized apremilast was added and the mixture was stirred until dissolved; the nonionic surfactant Tween 80 was added to 1/3 volume of the solvent (water), the mixture was evenly dispersed and then added to the above solution, the system was well stirred, then the solvent (water) was added to reach the total volume, and the buffer was added to adjust the pH to 5.0 to give the formulation.

The micronization process in the preparation process is air jet mixing;
the feed pressure of air jet milling is 6 bar, and the discharge pressure of air jet milling is 6 bar.

### Example 3

The inhalation aerosol of this example comprises an active drug component, a hydrofluoroalkane propellant, a polyethylene glycol surfactant, and a co-solvent.

The active drug is crisaborole, accounting for 2% w/w of the total amount of the formulation;
the propellant is 1,1,1,2-tetrafluoroethane, accounting for 96% w/w of the total amount of the formulation;
the co-solvent is ethanol, accounting for 1% w/w of the propellant.

The polyethylene glycol surfactant is PEG1000, accounting for 1% w/w of the total amount of the formulation.

The method for preparing the crisaborole inhalation aerosol is as follows:

The micronized active component crisaborole was added to an aluminum can, the system was weighed, the surfactant PEG1000 and the co-solvent ethanol were added, the aluminum can was quickly sealed with a metering valve, the propellant 1,1,1,2-tetrafluoroethane was added through the metering valve, and then the filled aerosol can was sonicated for 30 s to give the inhalation aerosol.

The micronization process in the preparation process is air jet mixing;
the feed pressure of air jet milling is 6 bar, and the discharge pressure of air jet milling is 6 bar.

### Example 4

The inhalation aerosol of this example comprises an active drug component, a hydrofluoroalkane propellant, a polyethylene glycol surfactant, and a co-solvent.

The active drug component is apremilast, accounting for 2% w/w of the total amount of the formulation;
the propellant is 1,1,1,2-tetrafluoroethane, accounting for 96% w/w of the total amount of the formulation;
the co-solvent is ethanol, accounting for 1% w/w of the propellant.

The polyethylene glycol surfactant is PEG1000, accounting for 1% w/w of the total amount of the formulation.

The method for preparing the apremilast inhalation aerosol is as follows:
The micronized active component apremilast was added to an aluminum can, the system was weighed, the surfactant PEG1000 and the co-solvent ethanol were added, the aluminum can was quickly sealed with a metering valve, the propellant 1,1,1,2-tetrafluoroethane was added through the metering valve, and then the filled aerosol can was sonicated for 30 s to give the inhalation aerosol.

The micronization process in the preparation process is air jet mixing;
the feed pressure of air jet milling is 6 bar, and the discharge pressure of air jet milling is 6 bar.

### Example 5

The dry powder inhalation formulation of this example comprises the following raw materials: an active drug and a carrier;
the active drug is roflumilast, accounting for 20% of the total amount of the formulation;
the carrier is lactose monohydrate, accounting for 80% of the total amount of the formulation;
the method for preparing the roflumilast dry powder inhalation formulation is as follows:
the active drug roflumilast was pulverized by air jet to give the first component, which was then mixed with the carrier lactose monohydrate to give a homogeneous mixture. After spheroidization, spherical particle samples were obtained and loaded into a Symbicort turbuhaler ^{®}.

In the roflumilast dry powder inhalation formulation, the feed pressure of air jet milling of the active drug is 6 bar, the discharge pressure of air jet milling is 6 bar, and the particle size is D90 4.4 µm; the carrier particle size is D90 7.2 µm;
the mixing method is air jet mixing, wherein the feed pressure of air jet milling is 1 bar, and the discharge pressure of air jet milling is 6 bar.

### Example 6

The dry powder inhalation formulation of this example comprises the following raw materials: an active drug and a carrier;
the active drug is apremilast, accounting for 20% of the total amount of the formulation;
the carrier is lactose monohydrate, accounting for 80% of the total amount of the formulation;
The method for preparing the apremilast dry powder inhalation formulation is as follows:
   The active drug apremilast was pulverized by air jet to give the first component, which was then mixed with the carrier lactose monohydrate to give a homogeneous mixture. After spheroidization, spherical particle samples were obtained and loaded into a Symbicort turbuhaler ^{®}.

In the apremilast dry powder inhalation formulation, the feed pressure of air jet milling of the active drug is 6 bar, the discharge pressure of air jet milling is 6 bar, and the particle size is D90 4.6 µm; the carrier particle size is D90 7.5 µm;
the mixing method is air jet mixing, wherein the feed pressure of air jet milling is 1 bar, and the discharge pressure of air jet milling is 6 bar.

### Example 7

The soft mist inhaler of this example comprises an active drug component, a solvent, an additive, and a pH adjuster.

The active drug component is apremilast, with a dose of 0.5 mg/mL;
the solvent is water;
the additive is EDTA, with a dose of 0.1 mg/mL.
the pH adjuster is citric acid, with a dose of 0.03 mg/mL;
the additive and citric acid were added to a beaker, a portion of the solvent was added to dissolve them, then micronized apremilast was added, water was added to make up to 200 mL, the mixture was well stirred and its pH was 3.94 as measured, the mixture was put into flexible plastic vials for later use.

The micronization process in the preparation process is air jet mixing;
the feed pressure of air jet milling is 6 bar, and the discharge pressure of air jet milling is 6 bar.

### Comparative Example 1

The difference between this comparative example and the example is that the formulation used is oral apremilast.

### Comparative Example 2

The difference between this comparative example and the example is that the formulation used is oral nintedanib.

### Experimental Example 1

According to the Pharmacopoeia of the People's Republic of China 2020, Volume IV for General Chapters 0951, based on the method for testing the aerodynamic properties of fine particles in inhalation formulations and using device 3, the inhalation formulations prepared in Examples 1-6 were tested. The test results are shown in Table 1.

**Table 1 Test results of fine particles (FPF) in Examples 1-7**

| Group | FPM (µg) | FPF (%) |
|---|---|---|
| Example 1 (Roflumilast aerosol inhalation solution) | 5.4 | 17.7 |
| Example 2 (Apremilast aerosol inhalation solution) | 5.8 | 18.2 |
| Example 3 (Crisaborole inhalation aerosol) | 36.8 | 29.4 |
| Example 4 (Apremilast inhalation aerosol) | 40.6 | 31.2 |
| Example 5 (Roflumilast dry powder inhaler) | 67.4 | 44.6 |
| Example 6 (Apremilast dry powder inhaler) | 69.1 | 46.8 |
| Example 7 (Apremilast soft mist inhaler) | 0.3 | 16.9 |

### Experimental Example 2

### Pharmacodynamic experiments

Establishment of pulmonary fibrosis model: 6-8 week old C57BL6J mice were selected and acclimatized for one week. In the model group, apremilast inhalation (Example 2) treatment group, roflumilast inhalation (Example 5) treatment group, crisaborole inhalation (Example 3) treatment group, apremilast oral administration treatment group (Comparative Example 1), and nintedanib oral administration treatment group (Comparative Example 2, nintedanib is a commonly used drug for treating IPF in the prior art), the animals were treated with bleomycin solution (3mg/kg/50µl) via intratracheal instillation on the first day, and in the control group, the animals were treated with an equal volume of physiological saline. Treatment started one week later and lasted for two weeks, and a total of three weeks was required. The bleomycin solution was prepared by mixing bleomycin dry powder with sterile PBS to reach the required concentration (non-surgical method of intratracheal injection of bleomycin into mouse lungs: the mice were anesthetized with isoflurane and suspended on a surgical stent at a 70° angle. Bleomycin was administered via intratracheal instillation using a 200 µl pipette).

The effects on lung injury in mice with pulmonary fibrosis in the apremilast inhalation treatment group, roflumilast inhalation treatment group, crisaborole inhalation treatment group, apremilast oral administration treatment group, and nintedanib oral administration treatment group were evaluated.

Weight measurement: The weight of mice was measured and recorded at a fixed time on the morning each week, and the weight changes of mice in each group at different times were observed.

HE staining and MASSON staining: Mouse lung tissue was fixed in 4% paraformaldehyde for 24 h, rinsed with running water overnight, dehydrated with alcohol conventionally, embedded in paraffin, cut into 5 µm sections, and stained with HE and MASSON respectively. After clearing with xylene, the sections were mounted with neutral resin. The pathological changes in the lungs were observed and photographed under an optical microscope.

Detection of malondialdehyde (MDA) and hydroxyproline (HYP) content in the lungs: An appropriate amount of lung tissue was taken from the mice in each group, homogenized (the ground tissue was centrifuged at 3000 rpm for 10 min and the supernatant was taken for later use), and the MDA and HYP content in the lungs was detected according to the kit instructions. MDA stands for malondialdehyde, a product of lipid peroxidation that reflects oxidative damage, while HYP stands for hydroxyproline level, which reflects the level of pulmonary fibrosis.

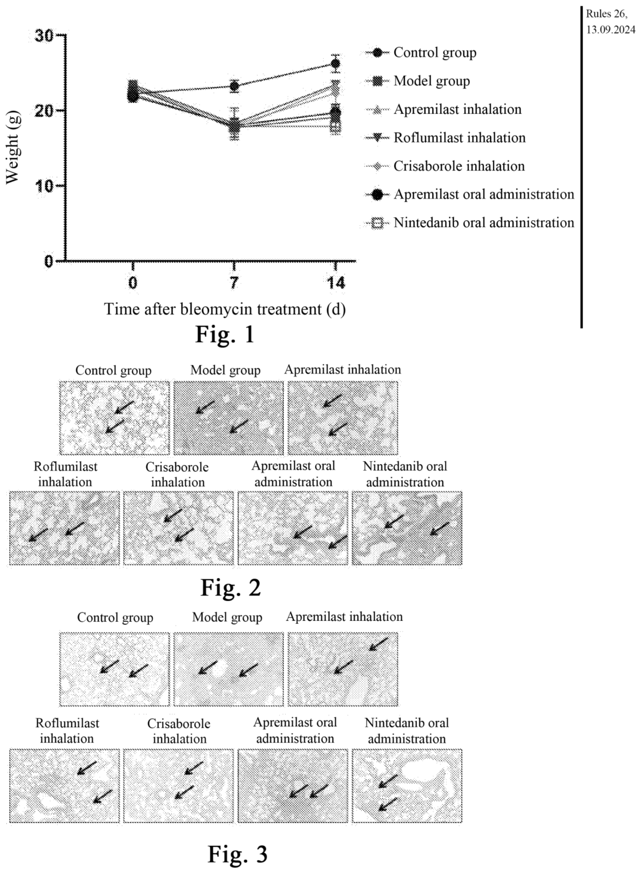

Statistical analysis: The results were statistically analyzed using Graphpad Prism 9.0 software for the experimental data of each group. All experimental data underwent normality test. The measurement data were expressed as mean ± standard deviation (mean ± SD). One-way ANOVA was used to compare the means of multiple groups of samples. P<0.05 is considered statistically significant. The results are shown in Figs. 1 to 4.

### 1) PDEi treatment can effectively improve bleomycin-induced weight loss in mice, and inhalation is more effective than oral administration

See Fig. 1: Compared with the control group, the mice treated with bleomycin for 1 week showed a significant decrease in body weight; compared with the model group, the mice in the apremilast inhalation treatment group, roflumilast inhalation treatment group, crisaborole inhalation treatment group, and apremilast oral administration treatment group showed significant weight recovery, while there was no significant difference in weight recovery in the nintedanib oral administration treatment group; compared with the apremilast oral administration treatment group, the mice in the apremilast inhalation treatment group, roflumilast inhalation treatment group, and crisaborole inhalation treatment group showed more significant weight recovery.

### 2) PDEi treatment reduces the pathological damage of pulmonary fibrosis, and inhalation is more effective than oral administration

As shown by MASSON staining in Fig. 2, compared with the control group, the model group showed significant pathological changes, with thickened alveolar interstitium and significantly increased collagen fiber deposition. Compared with the model group, the mice in the apremilast inhalation treatment group, roflumilast inhalation treatment group, and crisaborole inhalation treatment group showed fewer pathological changes in the lungs, with thinner alveolar interstitium and clearer boundaries between the parts; compared with the inhalation groups, the apremilast and nintedanib oral administration groups showed slightly thickened alveolar interstitium and a few visible collagen fibers (see arrows in Fig. 2), the arrows in the figure indicate collagen deposition, and as shown in Fig. 2: the control group had no collagen fiber deposition in the lungs; the model group had significantly increased collagen fibers in the lungs; the apremilast, roflumilast, and crisaborole inhalation groups showed a significant reduction in collagen fibers compared with the model group; and the apremilast and nintedanib oral administration groups showed reduced collagen deposition compared with the model group, but the degree of reduction was less than that in the inhalation groups.

As shown by HE staining in Fig. 3, compared with the control group, the mice in the model group had abnormal lung tissue structure, with significant thickening of the alveolar walls, unclear alveolar structure, obvious fibrosis, and obvious inflammatory cell infiltration. Compared with the model, the abnormal lung tissue structure of mice in the apremilast inhalation treatment group, roflumilast inhalation treatment group, and crisaborole inhalation treatment group was improved, with some alveoli filled with air, alveolar walls thinned, and very few inflammatory cells visible. Compared with the inhalation groups, the alveolar structure of mice in the oral apremilast and nintedanib groups was improved, with slightly thickened alveolar walls, some alveolar structures unclear, and inflammatory cell infiltration visible (see arrows in Fig. 3). The arrows in the figure indicate the alveolar structure and inflammatory cell infiltration. As shown in Fig. 3: the alveolar structure of the control group was clearly visible, with no inflammatory cell infiltration; in the model group, the lungs were dense, the alveolar cavity structure disappeared, and there was a large number of inflammatory cell infiltrations; the alveolar structure in the apremilast, roflumilast, and crisaborole inhalation groups was significantly improved compared with the model group, with no obvious inflammatory cell infiltration; the oral apremilast and nintedanib groups both had improved alveolar structure compared with the model group, with a few inflammatory cell infiltrations.

### 3) Inhaled PDEi can effectively reduce the content of MDA and HYP in the lungs

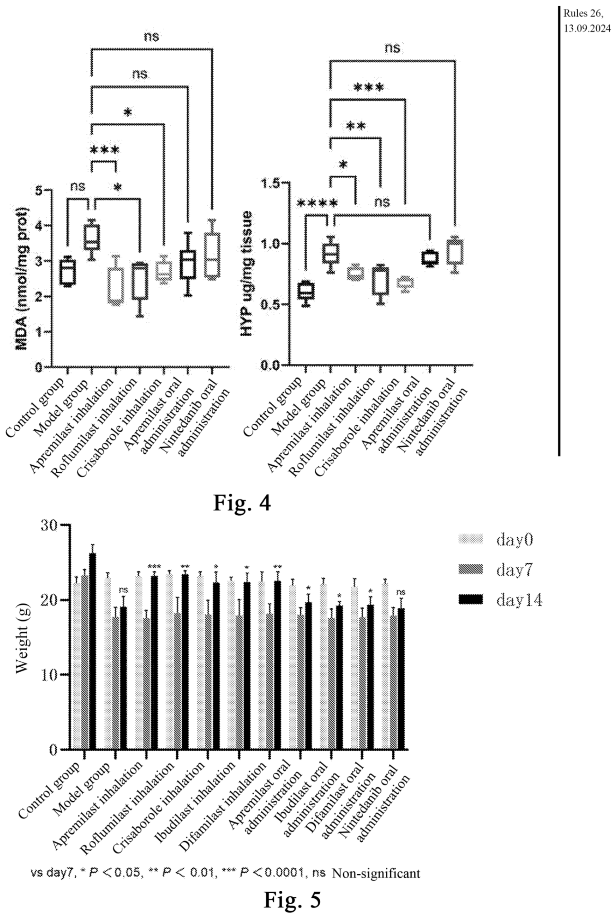

As shown in Fig. 4, compared with the control group, the levels of MDA and HYP in the lungs of the model group were significantly increased, and significantly decreased after inhalation treatment. Oral apremilast and nintedanib showed a slight tendency to alleviate MDA and HYP in the lungs, but the results were not statistically significant. The results indicate that apremilast inhalation treatment, roflumilast inhalation treatment, and crisaborole inhalation treatment can effectively reduce the oxidative stress level and collagen deposition in pulmonary fibrosis.

Nintedanib is a commonly used drug for treating IPF in the prior art, but due to its limited efficacy and significant systemic side effects, there is a large vacancy in the selection of drugs for the treatment of IPF. In summary, the present invention demonstrates, through parallel comparison of the efficacy of oral nintedanib in treating IPF, that drugs such as apremilast, roflumilast, and crisaborole exhibit superior therapeutic effects, and is therefore inventive.

The following are other examples of the present invention and their further description:

### Example 7

The soft mist inhaler of this example comprises an active drug component, a solvent, an additive, and a pH adjuster.

The active drug component is apremilast, with a dose of 0.5 mg/mL;
the solvent is water;
the additive is EDTA, with a dose of 0.1 mg/mL.
the pH adjuster is citric acid, with a dose of 0.03 mg/mL;
the additive and citric acid were added to a beaker, a portion of the solvent was added to dissolve them, then micronized apremilast was added, water was added to make up to 200 mL, the mixture was well stirred and its pH was 3.94 as measured, the mixture was put into flexible plastic vials for later use.

The micronization process in the preparation process is air jet mixing;
the feed pressure of air jet milling is 6 bar, and the discharge pressure of air jet milling is 6 bar.

### Example 8

The inhalation aerosol of this example comprises an active drug component, a hydrofluoroalkane propellant, a polyethylene glycol surfactant, and a co-solvent.

The active drug component is ibudilast, accounting for 2% w/w of the total amount of the formulation;
the propellant is 1,1,1,2-tetrafluoroethane, accounting for 96% w/w of the total amount of the formulation;
the co-solvent is ethanol, accounting for 1% w/w of the propellant.

The polyethylene glycol surfactant is PEG1000, accounting for 1% w/w of the total amount of the formulation.

The method for preparing the ibudilast inhalation aerosol is as follows:
The micronized active component ibudilast was added to an aluminum can, the system was weighed, the surfactant PEG1000 and the co-solvent ethanol were added, the aluminum can was quickly sealed with a metering valve, the propellant 1,1,1,2-tetrafluoroethane was added through the metering valve, and then the filled aerosol can was sonicated for 30 s to give the inhalation aerosol.

The micronization process in the preparation process is air jet mixing;
the feed pressure of air jet milling is 6 bar, and the discharge pressure of air jet milling is 6 bar.

### Example 9

The dry powder inhalation formulation of this example comprises the following raw materials: an active drug and a carrier;
the active drug is difamilast, accounting for 20% of the total amount of the formulation;
the carrier is lactose monohydrate, accounting for 80% of the total amount of the formulation;
the method for preparing the difamilast dry powder inhalation formulation is as follows:
the active drug difamilast was pulverized by air jet to give the first component, which was then mixed with the carrier lactose monohydrate to give a homogeneous mixture. After spheroidization, spherical particle samples were obtained and loaded into a Symbicort turbuhaler ^{®}.

In the difamilast dry powder inhalation formulation, the feed pressure of air jet milling of the active drug is 6 bar, the discharge pressure of air jet milling is 6 bar, and the particle size is D90 4.4 µm; the carrier particle size is D90 7.2 µm;
the mixing method is air jet mixing, wherein the feed pressure of air jet milling is 1 bar, and the discharge pressure of air jet milling is 6 bar.

### Example 10

The dry powder inhalation formulation of this example comprises the following raw materials: an active drug and a carrier;
the active drug is ibudilast, accounting for 20% of the total amount of the formulation;
the carrier is lactose monohydrate, accounting for 80% of the total amount of the formulation;
the method for preparing the ibudilast dry powder inhalation formulation is as follows:
the active drug ibudilast was pulverized by air jet to give the first component, which was then mixed with the carrier lactose monohydrate to give a homogeneous mixture. After spheroidization, spherical particle samples were obtained and loaded into a Symbicort turbuhaler ^{®}.

In the ibudilast dry powder inhalation formulation, the feed pressure of air jet milling of the active drug is 6 bar, the discharge pressure of air jet milling is 6 bar, and the particle size is D90 4.4 µm; the carrier particle size is D90 7.2 µm;
the mixing method is air jet mixing, wherein the feed pressure of air jet milling is 1 bar, and the discharge pressure of air jet milling is 6 bar.

### Experimental Example 3, conducted following the method described in Experimental Example 1 above

According to the Pharmacopoeia of the People's Republic of China 2020, Volume IV for General Chapters 0951, based on the method for testing the aerodynamic properties of fine particles in inhalation formulations and using device 3, the inhalation formulations prepared in Examples 8-10 were tested. The test results are shown in Table 2.

**Table 2 Test results of fine particles (FPF) in Examples 8-10**

| Group | FPM (µg) | FPF (%) |
|---|---|---|
| Example 8 (Ibudilast inhalation aerosol) | 42.6 | 33.5 |
| Example 9 (Difamilast dry powder inhaler) | 62.1 | 41.4 |
| Example 10 (Ibudilast dry powder inhaler) | 61.4 | 43.6 |

In addition to the aforementioned comparative examples 1 and 2, comparative examples 3 and 4 are added as follows:
Comparative Example 3
   The difference between this comparative example and the example is that the formulation used is oral ibudilast.
Comparative Example 4
   The difference between this comparative example and the example is that the formulation used is oral difamilast.

Furthermore, Experimental Example 4 was conducted based on the aforementioned Experimental Example 2.

### Pharmacodynamic experiments

Establishment of pulmonary fibrosis model: 6-8 week old C57/BL6J mice were selected and acclimatized for one week. The mice were divided into blank control group, model group, apremilast inhalation (Example 2) treatment group, roflumilast inhalation (Example 5) treatment group, crisaborole inhalation (Example 3) treatment group, ibudilast inhalation (Example 10) treatment group, difamilast inhalation (Example 9) treatment group, ibudilast oral administration (Comparative Example 3) treatment group, difamilast oral administration (Comparative Example 4) treatment group, and nintedanib oral administration (Comparative Example 2, nintedanib is a commonly used drug for treating IPF in the prior art) treatment group, with n = 8 in each group. On the first day, the mice in the model group and the treatment groups were administered bleomycin solution (3 mg/kg/50 µl) via tracheal instillation, while the control group was treated with an equal volume of physiological saline. Treatment began in each treatment group one week later and lasted for two weeks, and a total of three weeks was required. The bleomycin solution was prepared by mixing bleomycin dry powder with sterile PBS to reach the required concentration (non-surgical method of intratracheal injection of bleomycin into mouse lungs: the mice were anesthetized by inhaling isoflurane, and suspended on a surgical stent at a 70° angle, and bleomycin was administered via intratracheal instillation using a 200 µl pipette).

The effects on lung injury in mice with pulmonary fibrosis in the apremilast inhalation treatment group, roflumilast inhalation treatment group, crisaborole inhalation treatment group, ibudilast inhalation treatment group, difamilast inhalation treatment group, apremilast oral administration treatment group, ibudilast oral administration treatment group, difamilast oral administration treatment group and nintedanib oral administration treatment group were evaluated.

Weight measurement: The weight of mice was measured and recorded at a fixed time on the morning each week, and the weight changes of mice in each group at different times were observed.

HE staining and MASSON staining: Mouse lung tissue was fixed in 4% paraformaldehyde for 24 h, rinsed with running water overnight, dehydrated with alcohol as usual, embedded in paraffin, then serially cut into sections (5 µm), and stained with HE and MASSON respectively. After clearing with xylene, the sections were mounted with neutral resin. The sections were observed and photographed under an optical microscope.

Detection of malondialdehyde (MDA) and hydroxyproline (HYP) content in the lungs: An appropriate amount of lung tissue was taken from the mice in each group, homogenized (the ground tissue was centrifuged at 3000 rpm for 10 min and the supernatant was taken for later use), and the MDA and HYP content in the lungs was detected according to the kit instructions (MDA stands for malondialdehyde, a product of lipid peroxidation that reflects oxidative damage; HYP (hydroxyproline) level reflects the degree of fibrotic deposition in the lungs).

Statistical analysis: The results were statistically analyzed using Graphpad Prism 9.0 software for the experimental data of each group. All experimental data underwent normality test. The measurement data were expressed as mean ± standard deviation (mean ± SD). One-way ANOVA was used to compare the means of multiple groups of samples. P<0.05 is considered statistically significant. The results are shown in Figs. 5 to 9.

### 1) PDEi treatment can effectively improve bleomycin-induced weight loss in mice, and inhalation is more effective than oral administration

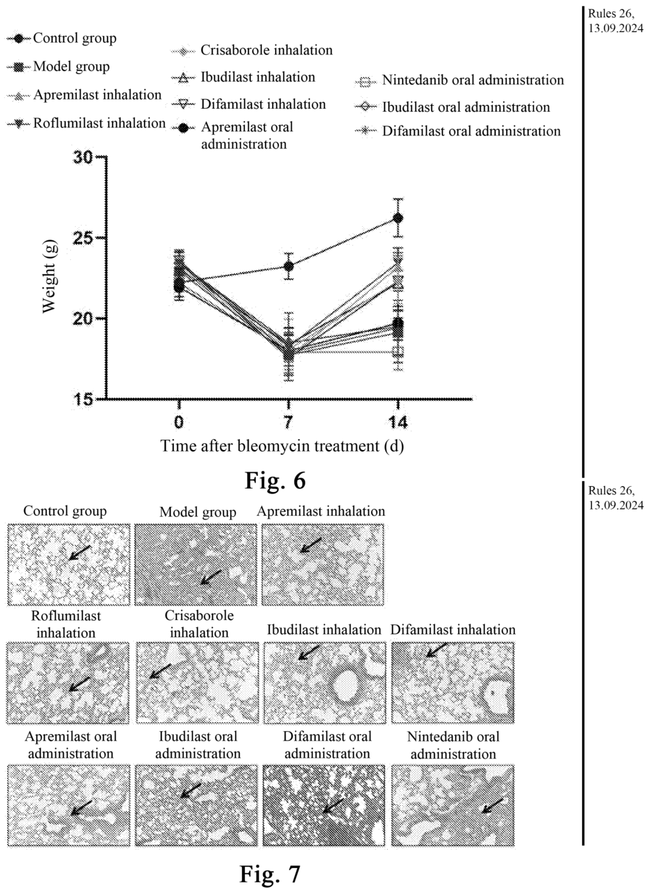

See Figs. 5 and 6: Compared with the control group, the mice treated with bleomycin for 1 week showed a significant decrease in body weight; compared with the model group, the mice in the apremilast inhalation treatment group, roflumilast inhalation treatment group, crisaborole inhalation treatment group, ibudilast inhalation treatment group, difamilast inhalation treatment group, apremilast oral administration treatment group, ibudilast oral administration treatment group and difamilast oral administration treatment group showed significant weight recovery, while there was no significant difference in weight recovery in the nintedanib oral administration treatment group compared with the model group; compared with the oral administration treatment groups, the mice in the drug inhalation treatment groups showed more significant weight recovery. As can be seen from Figs. 5 and 6, compared to the 7th day after the model establishment, the body weight of the mice in the model group did not show any significant recovery on the 14th day, while the mice in the apremilast inhalation treatment group, roflumilast inhalation treatment group, crisaborole inhalation treatment group, ibudilast inhalation treatment group, difamilast inhalation treatment group, apremilast oral administration treatment group, ibudilast oral administration treatment group, and difamilast oral administration treatment group had significant weight recovery, and the mice in the nintedanib oral administration treatment group did not show significant body recovery.

### 2) PDEi treatment reduces the pathological damage of pulmonary fibrosis, and inhalation is more effective than oral administration

As shown by MASSON staining in Fig. 7, compared with the control group, the model group showed significant pathological changes, with thickened alveolar interstitium, significantly increased collagen fiber deposition, and disappearance of the normal alveolar ventilation structure; compared with the model group, the mice in the apremilast inhalation treatment group, roflumilast inhalation treatment group, crisaborole inhalation treatment group, ibudilast inhalation treatment group, and difamilast inhalation treatment group had significantly less lung damage, with no thickening of the alveolar interstitium and with clear alveolar structure, which was similar to the control group; compared with the inhalation groups, the apremilast oral administration treatment group, ibudilast oral administration treatment group, difamilast oral administration treatment group and nintedanib oral administration treatment group showed slightly thickened alveolar interstitium and a few visible collagen fibers (see arrows in Fig. 7), the arrows in the figure indicate collagen deposition, and as shown in Fig. 7: the control group had no collagen fiber deposition in the lungs; the model group had significantly increased collagen fibers in the lungs; the apremilast, roflumilast, crisaborole, ibudilast and difamilast inhalation groups showed a significant reduction in collagen fibers compared with the model group; and the oral apremilast, ibudilast, difamilast and nintedanib showed reduced collagen deposition compared with the model group, but the degree of reduction was less than that in the inhalation groups.

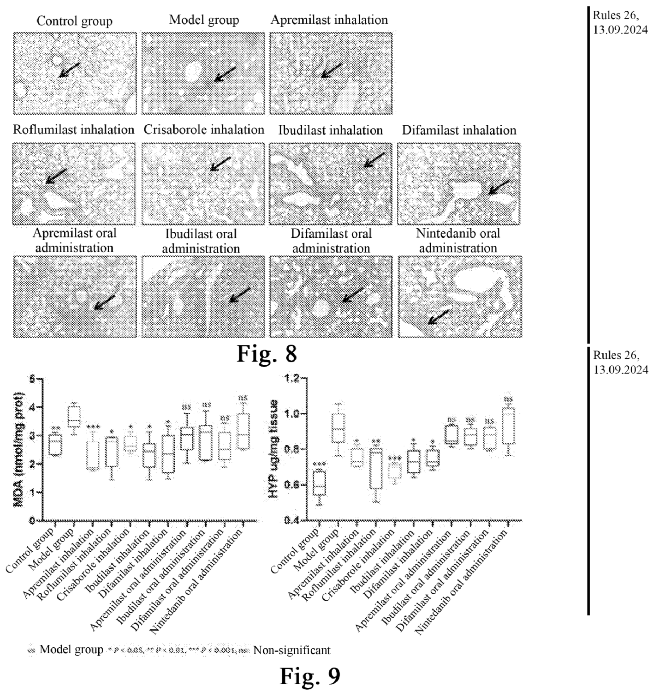

As shown by HE staining in Fig. 8, compared with the control group, the mice in the model group had abnormal lung tissue structure, with significant thickening of the alveolar walls, unclear alveolar structure, obvious fibrosis, and obvious inflammatory cell infiltration. Compared with the model, the mice in the apremilast inhalation treatment group, roflumilast inhalation treatment group, crisaborole inhalation treatment group, ibudilast inhalation treatment group, and difamilast inhalation treatment group had significantly improved lung tissue structure, with some alveoli filled with air, no obvious thickening of alveolar walls, and less infiltration of inflammatory cells. Compared with the inhalation treatment groups, the mice in the apremilast oral administration treatment group, ibudilast oral administration treatment group, difamilast oral administration treatment group and nintedanib oral administration treatment group had improved alveolar structure, with slightly thickened alveolar walls, some alveolar structures unclear, and inflammatory cell infiltration visible (see arrows in Fig. 8). The arrows in the figure indicate the alveolar structure and inflammatory cell infiltration. As shown in Fig. 8: the alveolar structure of the control group was clearly visible, with no inflammatory cell infiltration; in the model group, the lungs were dense, the alveolar cavity structure disappeared, and there was a large number of inflammatory cell infiltrations; compared with the model group, the alveolar structure in the apremilast, roflumilast, crisaborole, ibudilast and difamilast inhalation groups was significantly improved, with no obvious inflammatory cell infiltration; compared with the model group, oral apremilast, ibudilast, difamilast and nintedanib all improved the alveolar structure, with a few inflammatory cell infiltrations.

### 3) Inhaled PDEi can effectively reduce the content of MDA and HYP in the lungs

As shown in Fig. 9, compared with the control group, the levels of MDA and HYP in the lungs of the model group were significantly increased, and significantly decreased after inhalation treatment. The levels of MDA and HYP in the lungs of the mice in the apremilast, ibudilast, difamilast and nintedanib oral administration treatment groups showed a slight downward trend, but this did not have statistical significance. The results indicate that apremilast inhalation treatment, roflumilast inhalation treatment, crisaborole inhalation treatment, ibudilast inhalation treatment and difamilast inhalation treatment can effectively reduce the oxidative stress level and collagen deposition in pulmonary fibrosis.

As described above, nintedanib is a commonly used drug for treating IPF in the prior art, but due to its limited efficacy and significant systemic side effects, there is a large vacancy in the selection of drugs for the treatment of IPF. The present invention demonstrates, through parallel comparison of the efficacy of oral nintedanib in treating IPF, that drugs such as apremilast, roflumilast, and crisaborole exhibit superior therapeutic effects, and is therefore inventive. That is, in the present invention, the active drug is any one of apremilast, ibudilast, roflumilast, crisaborole, and difamilast; and the active drug includes, but is not limited to, apremilast, ibudilast, roflumilast, crisaborole, and difamilast.

In conclusion, the inhalation formulations for treating an IPF diseases and preparation method therefor provided by the present invention feature simple production process, portability, and the ability to effectively enhance the bioavailability of the active ingredients.

It will be apparent to those skilled in the art that the present invention is not limited to the details of the exemplary embodiments described above, and that the present invention can be implemented in other specific forms without departing from the spirit or essential characteristics of the present invention. Therefore, the embodiments should be regarded as exemplary and nonlimiting in all respects, and the scope of the present invention is defined by the appended claims rather than the foregoing description. Thus, it is intended that all variations falling within the implications and scope of the equivalents of the claims be included within the present invention.

Furthermore, it should be understood that although the specification describes embodiments, not every embodiment contains only one independent technical solution. This narrative style of the specification is merely for clarity. Those skilled in the art should consider the specification as a whole, and the technical solutions in the embodiments can also be appropriately combined to form other embodiments that can be understood by those skilled in the art.

## Claims

1. An inhalation formulation for treating an IPF disease, wherein the inhalation formulation is an inhalation liquid formulation, an inhalation aerosol, a dry powder inhaler, or a soft mist inhaler;
the inhalation formulation contains an active drug, and the active drug is any one of apremilast, ibudilast, crisaborole, and difamilast.

2. The inhalation formulation according to claim 1, wherein the inhalation formulation is an inhalation liquid formulation.

3. The inhalation formulation according to claim 2, wherein the aerosol inhalation liquid formulation consists of an active drug and a pharmaceutical excipient A;
the pharmaceutical excipient A includes a solvent, a nonionic surfactant, a pH buffers, an osmotic pressure regulator, and a complexing agent.

4. The inhalation formulation according to claim 3, wherein the solvent is deionized water;
the nonionic surfactant is any one of Tween, Span, polyol, and poloxamer;
the pH buffer is any one of acetic acid-sodium acetate, phosphate-sodium phosphate, and citrate-sodium citrate, with a pH value of 4.0-6.0;
the osmotic pressure regulator is any one of sodium chloride, propylene glycol, glycerol, and glucose;
the complexing agent is EDTA or a salt thereof.

5. A method for preparing the inhalation formulation according to any one of claims 2 to 4, comprising the following preparation steps:
step S1. dissolving the osmotic pressure regulator and complexing agent in the solvent, stirring the mixture until completely dissolved, adding the active drug, and stirring the mixture until completely dissolved to give a solution A;
step S2. adding the solvent to the nonionic surfactant and well dispersing the system to give a solution B;
step S3. adding the solution B to the solution A, stirring the mixture well, then adding the remaining solvent, and adding the pH buffer to adjust the pH to give an inhalation aerosol.

6. The inhalation formulation according to claim 1, wherein the inhalation formulation is an inhalation aerosol.

7. The inhalation formulation according to claim 6, wherein the inhalation aerosol consists of an active drug component and a pharmaceutical excipient B;
the pharmaceutical excipient B includes a hydrofluoroalkane propellant, a polyethylene glycol surfactant, and a co-solvent.

8. The inhalation formulation according to claim 7, wherein the active drug component is prepared by micronization, wherein micronization is any one of air jet milling, spray drying, spray freeze drying, recrystallization, and ball milling.

9. The inhalation formulation according to claim 7, wherein the hydrofluoroalkane propellant is 1,1,1,2,3,3,3-heptafluoropropane or 1,1,1,2-tetrafluoroethane;
the polyethylene glycol is any one of PEG200, PEG400, and PEG1000;
the co-solvent is propylene glycol or ethanol.

10. A method for preparing the inhalation formulation according to any one of claims 6 to 9, wherein the micronized active drug component is added to an aluminum can, the system is weighed, the surfactant and the co-solvent are added, the aluminum can is quickly sealed with a metering valve, the propellant is added through the metering valve, and then the filled aerosol can is sonicated for 30 s to give an inhalation aerosol.

11. The inhalation formulation according to claim 1, wherein the inhalation formulation is a dry powder inhaler.

12. The inhalation formulation according to claim 11, wherein the dry powder inhaler consists of an active drug and a carrier;
the carrier is at least one of lactose, mannose, chitosan, DPPC, and DSPC.

13. The inhalation formulation according to claim 12, wherein the content ratio of the active drug to the carrier is (5-100):(1-95).

14. The inhalation formulation according to claim 12, wherein the particle size D90 of the active drug is 1-10 µm.
the particle size D90 of the carrier is 3-200 µm.

15. A method for preparing the inhalation formulation according to any one of claims 11 to 14, comprising the following steps:
S01. micronizing the active drug to give a first component;
S02. mixing the first component with the carrier to give a second component;
S03. preparing the second component into a dry powder formulation and loading it into an appropriate device for later use.

16. The method for preparing the inhalation formulation according to claim 15, wherein micronization is any one of air jet milling, spray drying, spray freeze drying, recrystallization, and ball milling;
the mixing method is any one of high-shear mixing, air jet mixing, and three-dimensional mixing.

17. The inhalation formulation according to claim 1, wherein the inhalation formulation is a soft mist inhaler.

18. The inhalation formulation according to claim 17, wherein the soft mist inhaler consists of an active drug and a pharmaceutical excipient C;
the pharmaceutical excipient C includes a solvent, an additive, and a pH adjuster.

19. The inhalation formulation according to claim 18, wherein the solvent is deionized water;
the additive is EDTA or a salt thereof;
the pH adjuster is citric acid, hydrochloric acid, or sodium hydroxide, and the pH range of the formulation is 2 to 6.

20. A method for preparing the inhalation formulation according to any one of claims 17 to 19, wherein the additive and pH adjuster are added to a beaker, a portion of the solvent is added to dissolve them, then the micronized active drug is added, and the solvent is replenished to reach the required concentration, the mixture is well stirred, then the pH of the mixture is measured, and the mixture is put into flexible plastic vials for later use.
